(19) Europäisches Patentamt

European Patent Office

Office européen des brevets

(11) **EP 1 772 767 A2**

(12) **EUROPEAN PATENT APPLICATION**

(43) Date of publication:
**11.04.2007 Bulletin 2007/15**

(51) Int Cl.:
*G02C 7/04* (2006.01)    *G02B 1/04* (2006.01)
*A61K 9/00* (2006.01)    *B29D 11/00* (2006.01)

(21) Application number: **06255173.4**

(22) Date of filing: **06.10.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **07.10.2005 US 246771**

(71) Applicant: **Johnson and Johnson Vision Care, Inc. Jacksonville, FL 32256 (US)**

(72) Inventors:
• **Powell, P. Mark**
 **Jacksonville, Florida 32258 (US)**
• **Rooney, Thomas R.**
 **Jacksonville, Florida 32256 (US)**
• **Grammer, Holly L.**
 **Jacksonville, Florida 32206 (US)**
• **Rathore, Osman**
 **Jacksonville, Florida 32256 (US)**
• **Martin, W. Anthony**
 **Orange Park, Florida 32073 (US)**
• **Raja, Ranganath**
 **jcksonville, Florida 32256 (US)**
• **Mahadevan, Shivkumar**
 **Orange Park, Florida 32203 (US)**

(74) Representative: **Fisher, Adrian John et al**
 **CARPMAELS & RANSFORD**
 **43-45 Bloomsbury Square**
 **London WC1A 2RA (GB)**

(54) **Method for forming contact lenses comprising therapeutic agents**

(57) The present invention relates to a process comprising (a) dosing a reactive mixture comprising at least one crosslinkable prepolymer into a mold; (b) curing said reactive mixture to form a medical device; (c) contacting, said medical device with no more than about 500 mL of solution per medical device; and (d) incorporating, prior to or during said contacting step (c), at least one therapeutic agent.

**EP 1 772 767 A2**

**Description**

RELATED APPLICATION INFORMATION

This patent application is a continuation in part of USSN 10/983,851, which is a divisional of US 6,846,892, which claimed priority of provisional application, U.S. Ser. No. 60/363,639 filed on March 11, 2002.

FIELD OF THE INVENTION

This invention relates to methods for forming contact lenses comprising therapeutic agents.

BACKGROUND OF THE INVENTION

Contact lenses have been used commercially to improve vision since the 1950s. Most current contact lenses are made of hydrogels formed by polymerizing hydrophilic monomers such as HEMA and vinylpyrrolidone in the presence of a minor amount of a crosslinking agent. The polymerization of the monomers results in shrinkage which may be as much as 20% by volume.
Prepolymers having backbones of PVA and reactive acrylate groups have been disclosed. The reactive prepolymer is dissolved in water, and crosslinked inside a mold by irradiation with UV light to form a contact lens. The shrinkage during cure is small, but the hydrogels thus produced exhibit mechanical properties that may prove marginal for contact lens use.

SUMMARY OF THE INVENTION

The present invention relates to a process comprising (a) dosing a reactive mixture comprising at least one crosslinkable prepolymer into a mold; (b) curing said reactive mixture to form a medical device; (c) Contacting said medical device with no more than about 500 mL of solution per device; and (d) incorporating, prior to or during said contacting step (c), at least one therapeutic agent.

DETAILED DESCRIPTION

The present invention relates to processes for the production of medical devices comprising at least one therapeutic agent. More specifically, the present invention relates to the production of contact lenses comprising at least one therapeutic agent from a reactive mixture comprising at least one prepolymer. It has been found that when the post cure processing of the medical device comprising the therapeutic agent is conducted using less than about 500 mL per lens of solution, the lens retains far more of the therapeutic agent than when more solution is used.
As used herein, a "medical device" is any article that is designed to be used while either in or on mammalian tissues or fluid, and preferably in or on human tissue or fluids. Examples of these devices include but are not limited to catheters, implants, stents, and ophthalmic devices such as intraocular lenses and contact lenses. The preferred biomedical devices are ophthalmic devices, particularly contact lenses, most particularly contact lenses made from silicone hydrogels.
As used herein, the terms "lens" and "ophthalmic device" refer to devices that reside in or on the eye. These devices can provide optical correction, wound care, drug delivery, diagnostic functionality, cosmetic enhancement or effect or a combination of these properties. The term lens includes but is not limited to soft contact lenses, hard contact lenses, intraocular lenses, overlay lenses, ocular inserts, and optical inserts.
As used herein, the term prepolymer means a compound comprising at least about 10 repeating monomeric units, a polydispersity (Pd) of less than about 3 and one or more cross linkable group covalently bound to the compound. Prepolymers useful in the present invention may have weight average molecular weights (Mw) of about 50 to about 150 kD. In some embodiments the Pd may be less than about 2, and in some cases between about 1.2 and about 1.8.
The prepolymer backbone may be formed from any monomer(s) which are suitable for the manufacture of contact lenses. Suitable monomers are known to those of skill in the art, and include, but are not limited to vinyl-containing monomers. The term "vinyl-type" or "vinyl-containing" monomers refer to monomers comprising the vinyl group (-CR=CR'R", in which R, R' and R" are monovalent substituents), which are known to polymerize relatively easily. Suitable vinyl-containing monomers include 2-hydroxyethyl methacrylate ("HEMA"), N,N-dimethyl acrylamide (DMA), glycerol methacrylate (GMA), 2-hydroxyethyl methacrylamide, polyethyleneglycol monomethacrylate, methacrylic acid (MAA), acrylic acid, N-vinyl lactams (e.g. N-vinylpyrrolidone, or NVP), N-vinyl-N-methyl acetamide, N-vinyl-N-ethyl acetamide, N-vinyl-N-ethyl formamide, N-vinyl formamide, vinyl carbonate monomers, vinyl carbamate monomers, siloxane containing compounds with polymerizable vinyl groups, oxazolone monomers mixtures thereof and the like. Any of the other monomers disclosed in US 6,846,892, the disclosure of which is incorporated by reference, may also be used.

Generally the cross linkable group provides the ability to crosslink and form crosslinked polymers or hydrogels from the prepolymer. Suitable reactants that provide the crosslinkable groups have the structure A-S-F, where A is an attaching group which is covalently bound to the prepolymer backbone; S is a spacer and F is a functional group comprising an ethylenically unsaturated moiety. Suitable attaching groups, A, include halides, isocyanates, acids, acid anhydrides, acid halides, epoxides, azalactones, combinations thereof and the like. Preferred attaching groups include acid anhydrides.

The spacer may be a direct bond, a straight, branched or cyclic alkyl or aryl group having 1 to 8 carbon atoms. In some embodiments the space is a 1 to 4 carbon alkyl group or a polyether chain of the formula $-(CH_2-CH_2-O)_n-$ where n is between 1 and 8 and in some embodiments between 1 and 4.

Suitable functional groups comprise free radical polymerizable ethylenically unsaturated moieties. Suitable ethylenically unsaturated groups have the formula

$$-C(R^{10})=CR^{11}R^{12}$$

Where $R^{10}$, $R^{11}$ and $R^{12}$ are independently selected from H, $C_{1-6}$ alkyl, carbonyl, aryl and halogen. Preferably $R^{10}$, $R^{11}$ and $R^{12}$ are independently selected from H, methyl, aryl and carbonyl, and more preferably in some embodiments selected from H and methyl.

Examples of suitable reactants include methacryloyl chloride, 2-isocyanatoethylacrylate, isocyanatoethyl methacrylate (IEM), glycidyl methacrylate, cinnamoyl chloride, methacrylic anhydride, acrylic anhydride, maleic anhydride and 2-vinyl-4-dimethylazalactone. Methacrylic anhydride is preferred.

Suitable amounts of the crosslinkable group attached to prepolymer backbone include from about 1 to about 70 %, in some embodiments between about 1 and about 20%, in some embodiments about 1.5 to about 10 %, in some embodiments from about 2 to about 5% and in other embodiments from about 1.8 to about 3% on a stoichiometric basis based upon the amount of available attaching groups in the prepolymer backbone. The degree of functionalization may be measured by known methods such as determination of unsaturated groups or by cleavage of a bond between the functional reactant and the polymer followed by determination of the released compound by HPLC.

Suitable prepolymers may be made using the processes disclosed in US 6,846,8921, US 6,407,145 and US 6,106,746. Examples of suitable prepolymers may be found in US 6,846,8921, WO03/003073 and US 6,407,145, the disclosures of these and all other patents and applications cited herein are incorporated by reference in their entirety.

The prepolymer is included in a reactive mixture. The reactive mixture comprises the prepolymer and any other components desired to form the medical device, ophthalmic device or contact lens. The process of forming a medical device according to the present invention will be discussed using a contact lens as an example. However, those of skill in the art, using the teachings of the present application, may make other medical devices. Suitable components include, but are not limited to, diluents, initiators, ultraviolet absorbing compounds, reactive dyes, organic and inorganic pigments, dyes, photochromic compounds, release agents, antimicrobial compounds, mold lubricants, wetting agents, other additives desirable to maintain a consistent product specification, combinations thereof and the like.

If a diluent is used, it should be capable of dissolving, at or below 65°C, between about 30 weight % to about 60 weight % prepolymer based upon the total weight of the reactive mixture. Specific examples include alcohols having one to four carbon atoms, and preferably methanol, ethanol, propanol and mixtures thereof.

In some embodiments it may be desirable to use a diluent which is also safe for the medical device's intended end use. So, for example, when the medical device being formed is a contact lens, the solvent should preferably be safe for ocular contact and ophthalmically compatible. This is particularly important for diluents that will not or will only partially be removed from the resulting article prior to use. Diluents that will not be evaporated from the resulting article should have the capability to bring the Tg of the viscous solution to below about room temperature, (preferably a Tg less than about -50°C) and low vapor pressures (boiling point above about 180°C). Examples of biocompatible diluents include polyethylene glycols, glycerol, propylene glycol, dipropylene glycol mixtures thereof and the like. Preferred polyethylene glycols have molecular weights between about 200 and 600. Use of biocompatible diluents allows the removal of a separate washing/evaporation step to remove the diluents.

Water may be used as a co-diluent in minor amounts such as less than about 50% of the total diluent. For hydrogels, diluents may be added to the prepolymer in an amount which is approximate or equal to the amount of water present in the final hydrogel. Diluent amounts between about 40 and about 70 weight % of the resulting reactive mixture are acceptable.

A therapeutic agent may also be incorporated into the reactive mixture. A wide variety of therapeutic agents may be used. Suitable therapeutic agents include those that treat or target any part of the ocular environment, including the anterior and posterior sections of the eye and include pharmaceutical agents, vitamins, nutraceuticals combinations thereof and the like. Suitable , including classes of pharmaceutical compounds include antihistamines, antibiotics, glaucoma medication, carbonic anhydrase inhibitors, anti-viral agents, anti-inflammatory agents, non-steroid anti-imflammatory drugs, antifungal drugs, anesthetic agents, miotics, mydriatics, immunosuppressive agents, antiparasitic drugs, antiprotozoal drugs, combinations thereof and the like. Specific examples of therapeutic agents include acycylovir, adrenalone, aminocaproic acid, amoxicillin, amotriphene, amoxecaine, amodiaquin, antazoline, atrophine, betaxolol, bimatoprost, bupivacaine, carbachol, carteolol, chlorampenicol, chlortetracycline, clemastine, corynathine, cromalyn sodium, cyclopentolate, demecarium, dexamethasone, dichlorphenamide, dibutoline, diclophenac, dipivefrin, ephedrine, erythromycin, ethambutol, eucatropine, fluoromethalone, gentamycin, gramicidin, homatropine, indomethacin, ketotifen, levallorphan, levobunolol, levocabastine, lidocaine, lignocaine, lomefloxacin, loratidine, medrysone, mepivacaine, methazolamide, naphazoline, natamycin, natamycin, neomycin, noradrenaline, ofloxacin, oxybuprocaine, oxymetazoline, pheniramine, phenylephrine, physostigmine, pilocarpine, polymyxin B, prednisolone, proparacaine, pyrilamine, scopolamine, sorbinil, sulfacetamide, tamoxifen, tetracaine, tetracycline, tetrahydozoline, timolol, trifluridine, tropicamide, vidarabine, and salts and mixtures thereof and the like. In another embodiment the therapeutic agent is selected from the group consisting of ketotifen fumarate, pheniramine maleate, clemastine, loratidine, 11-dihydro-11-(1-methyl-4-piperidinylidene)-5H-imidazo[2,1-b][3]benzazepine-3-carboxaldehyde (CAS# 147084-10-4, olapatadine and mixtures thereof. In yet another embodiment the therapeutic agent comprises ketotifen fumarate, nor ketotifen fumarate, ,11-dihydro-11-(1-methyl-4-piperidinylidene)-5H-imidazo[2,1-b][3]benzazepine-3-carboxaldehyde (CAS# 147084-10-4, olapatadine, bimatoprost and mixtures thereof. Examples of nutriceutical compounds include vitamins and supplements such as vitamins A, D, E, lutein, zeaxanthin, lipoic acid, flavonoids, ophthalmicially compatible fatty acids, such as omega 3 and omega 6 fatty acids, combinations thereof, combinations with pharmaceutical compounds and the like. In one embodiment the therapeutic agent and prepolymer are selected such that there is a chemical or physical interaction or affinity between them. Such interactions include ionic bonding, hydrogen bonding, hydrophobic and hydrophilic interactions and the like. In one embodiment the therapeutic agent is water soluble. As used herein, water soluble means that the selected therapeutic agent does form visible to the human eye precipitates or gel particles at the concentrations selected and across the temperatures and pH regimes common for manufacturing, sterilizing and storing contact lenses.

A polymerization initiator may also be added. The initiator may be any initiator that is active at the processing conditions. Suitable initiators include thermally activated, photoinitiators (including UV and visible light initiators) and the like. Suitable thermally activated initiators include lauryl peroxide, benzoyl peroxide, isopropyl percarbonate, azobisisobutyronitrile, 2,2-azobis isobutyronitrile, 2,2-azobis 2-methylbutyronitrile and the like. Suitable photoinitiators include aromatic alpha hydroxyketones or a tertiary amine plus a diketone. Illustrative examples of photoinitiator systems are 1-hydroxycyclohexylphenyl ketone, 2-hydroxy-methyl-1-phenyl-propan-1-one, benzophenone, thioxanthen-9-one, a combination of camphorquinone and ethyl-4-(N,N-dimethylamino)benzoate or N-methyldiethanolamine, hydroxycyclohexyl phenyl ketone, bis(2,4,6-trimethylbenzoyl)-phenyl phosphine oxide and bis(2,6-dimethoxybenzoyl)-2,4,4-trimethylpentyl phosphine oxide, (2,4,6-trimethylbenzoyl)diphenyl phosphine oxide and combinations thereof and the like. Photoinitiation is a preferred method and bis(2,6-dimethoxybenzoyl)-2, 4, 4-trimethylpentyl phosphine oxide, bis(2,4,6-trimethylbenzoyl)-phenyl phosphine oxide and 2-hydroxy-methyl-1-phenyl-propan-1-one are preferred photoinitiators. Other initiators are known in the art, such as those disclosed in US 5,849,841, at column 16, the disclosure of which is incorporated herein by reference.

A lens forming amount of the reactive mixture is dosed into a mold having the shape of the final contact lens. Any mold material may be used so long as the mold material does not react or dissolve with the reactive mixture. Suitable non-limiting examples of mold materials, include polystyrene, polypropylene, cyclic polyolefins such as Topas® (cyclic olefin copolymer, commercially available from Ticona), Zeonor® (amorphous cycloolefin polymer, commercially available from Zeon), polycarbonate, polysulfone, polyethersulfone, polytetrafloroethylene (Teflon) or other floropolymers . In one embodiment the mold comprises two parts a front mold half and a back mold half. In another embodiment, the either the front or back mold half may be used as the contact lens package. In some embodiments either the front or back mold half may be reusable. In some embodiments one mold half is used as the contact lens package and the other mold half is reusable.

By "lens-forming amount" is meant an amount sufficient to produce a lens of the size and thickness desired. Typically, about 10 to about 50 $\mu$l of viscous solution is used per contact lens. In one embodiment the amount of reactive mixture dosed is equal to the amount needed to form the contact lens. Exact dosing in this manner insures that no extra polymer is left after polymerization. This reduces the amount of waste that must be handled.

The mold containing the reactive mixture is cured, such as by exposure to ionizing or actinic radiation, for example electron beams, X-rays, UV or visible light, ie. electromagnetic radiation or particle radiation having a wavelength in the range of from about 280 to about 650 nm. Also suitable are UV lamps, He/Cd , argon ion or nitrogen or metal vapor or NdYAG laser beams with multiplied frequency or light emitting diodes (LED). The selection of the radiation source and initiator are known to those of skill in the art. Those of skill in the art will also appreciate that the depth of penetration of the radiation in to the viscous solution and the crosslinking rate are in direct correlation with the molecular absorption coefficient and concentration of the selected photoinitiator. In a preferred embodiment the radiation source is selected from UVA (about 315 - about 400 nm), UVB (about 280-about 315) or visible light (about 400 -about 450 nm), at high intensity. As used herein the term "high intensity" means those between about 100 mW/cm$^2$ to about 10,000

mW/cm2. The cure time is short, generally less than about 30 seconds and preferably less than about 10 seconds. The cure temperature may range from about ambient to elevated temperatures of about 90°C. For convenience and simplicity the curing is preferably conducted at about ambient temperature. The precise conditions will depend upon the components of lens material selected and are within the skill of one of ordinary skill in the art to determine.

The cure conditions must be sufficient to form a polymer network from the crosslinkable prepolymer. The resulting polymer network is swollen with the diluent and has the form of the mold cavity.

Once curing is completed, the molds are opened. In embodiments where the therapeutic agent has been included in the reactive mixture, the cumulative amount of solution used in post cure processing steps is less than about 500 mL per lens, in some cases less than 100 mL per lens, in other embodiments between about 0.005 to about 100 mL in other embodiments between about 0.1 to about 50 mL per lens, in other embodiments between about 0.5 to about 10 mL per lens and in other embodiments between about 0.5 to about 5 mL per lens. As used herein, post cure processing steps include the steps between curing the lens and sealing the lens package containing the lens, and include contacting the lens with a solution to help release the lens from the lens mold (lens release), contacting the lens with a solution to remove unpolymerized components, byproducts and the like (extraction), and contacting the lens with a solution to swell the lens to it final desired size (hydration). In one embodiment the amount of solution used in the contacting step is limited to the amount of solution necessary hydrate the lens. Depending upon the size of the bowl of the lens package and the amount of packing solution desired, as little as 0.005 to 0.5 mL can be used in this embodiment.

In other embodiments the therapeutic agent may be incorporated in the hydrating solution. In these embodiments, the lenses may be extracted using conventional conditions and then hydrated as described above.

The hydrating solution should be compatible with the human eye, and in some embodiments, should be suitable for packaging and storing contact lenses. Examples include, deionized water and saline solutions, such as borate buffered saline solution.

In other embodiment the therapeutic agent may be included in the hydrating solution.

The resulting lenses comprise a therapeutic effective amount of at least one therapeutic agent. Therapeutic effective amount means an amount of therapeutic agent necessary to provide a desired benefit. For example, if the therapeutic agent is an anti-allergen, a therapeutic effective amount would be an amount sufficient to provide the wearer with relief from eye allergy symptoms such as itchy eyes or swollen eyelids. The therapeutic effective amount will vary for each therapeutic agent.

The hydrated contact lens may be sterilized by any means such as autoclaving, exposure to microwave or UV irradiation, chemical sterilization, asceptic packaging and the like.

In the present application all molecular weights are to be understood as molecular weights determined by the gel permeation chromatography (GPC) analysis (also called Size Exclusion Chromatography) using the method developed by K. Almdal of the Risø National Laboratories, Denmark (Almdal, K., Absolute Molar Mass Distribution Determination by Size Exclusion Chromatography. Synthesis of Narrow Molar Mass Distribution Polymers. Characterization of the Molar Mass Distribution of Poly(2-Hydroxyethyl Methacrylate) by Size Exclusion Chromatography with Coupled Refractive Index and Low Angle Laser Light Scattering Detection. Risø-M-2787(v.1) (1989) 141 p).

In this method a range of polyethylene glycols and polyethylene oxides with well defined molecular weights are used in the calibration of the equipment. These standards used for p-HEMA give more accurate values for peak molecular wt and Pd than previous methods developed for more hydrophobic polymers. The method is described below.

Molecular weight may be measured as follows. The SEC equipment is composed of a column oven at 40°C, a PE LC-410 pump with PE Nelson 900A/D and a series 200 autosampler. The detector is a RI Merck L7490.

The column combination consists of two TSK-Gel columns from TosoHaas (G4000PW + G2500PW) and a guardcolumn.

The eluent is made with methanol-water (75/25 wt/wt) and adjusted to 50 mM sodium chloride (NaCl).

The flow rate is 0.5 mL/minute. The injection volume is 150 $\mu$L and the run time is 60 minutes.

The calibration curve is obtained with third order regression using PEG and PEO of Peak molecular weights ranging from 960000 to 194 as standard references. These polymer standards are purchased from Polymer Laboratories Inc, Amherst MA. (Calibration kits PEG-10 part No 2070-0100; PEO-10 part No 2080-0101). Added standard reference PEG of Peak molecular weight of 194 gives a flow signal at a well-defined position, which is used as an internal standard or fixation point. Added NaCl plays the same role and gives a second fixation point.

Peak integrations are manually made. Integration start and end points are manually determined from significant difference on global baseline. Result reports give Mz, Mw, Mn, and Mpeak. in PEG/PEO units. Related values in HEMA units are calculated from the standard report with the following mathematical function:

$$M_{HEMA} = 10.^{1,362+0,7854*\log M, PEG/PEO}$$

The injection solutions are prepared with methanol-water 75/25 wt/wt adjusted to 60 mM NaCl to give a polymer concentration of 2mg/mL. Tetraethylene glycol is added to the sample in a concentration of 1 mg/ml in order to give a peak flow reference. The solutions are filtered on $0.5\mu m$ disposable filters before the injection is performed.

In the present invention polydispersity, Pd of a polymer sample is defined as Pd = $M_w$ / $M_n$ The peak molecular weight Mp is the molecular weight of the highest peak in the molecular weight distribution curve.

The following examples do not limit the invention. They are meant only to suggest a method of practicing the invention. Those knowledgeable in the field of contact lenses as well as other specialties may find other methods of practicing the invention. However, those methods are deemed to be within the scope of this invention.

Example 1

Prepolymers of hydroxyethyl methacrylate (HEMA) and methacrylic acid (MAA) having weight average molecular weights of 117 kD and 171 kD having a Pd of 1.7 and 1.6 respectively, and an average degree of functionality of 3.0% was made as described below.

Backbone polymerization

A 5 L jacketed 3-neck reaction flask was equipped with a graham condenser maintained at 5° C and an IKA® Eurostar power control-visc overhead mechanical stirrer. The flask was flushed with a stream of nitrogen for 15 minutes. A circulating temperature controller was preheated to 90° C. The following solids were then weighed and added via the unused neck using a plastic funnel: 1.650 g of AIBN (reagent grade, Aldrich), 3.000 g of Norbloc®, 0.036 g of blue HEMA (the reaction product of Reactive Blue 4 and HEMA, as described in Example 4 of U.S. Pat. no. 5,944,853). The following liquids were next added: 6.300 g of MAA (reagent grade, Aldrich), 300 $\mu$L of TMPTMA (trimethylolpropane trimethacrylate, from Esstech), 300.0 g of HEMA (from Rhom). The two beakers used to weigh the reagents were each rinsed with 3 x 100 mL of anhydrous reagent grade ethanol (VWR). An additional 2900 mL of anhydrous reagent grade ethanol along with the combined rinsings were added to the flask using a plastic funnel. The mixture was then stirred at 200 rpm for 20 minutes while being degassed with a steady stream of nitrogen passed through a polyethylene frit. After 20 minutes a nitrogen stream was introduced through the condenser, the fritted nitrogen line was removed and the unused neck was stoppered with a #49 suba seal. The reaction was kept under a small positive nitrogen pressure by continuously venting nitrogen through a mineral oil bubbler. The flask was next connected to the temperature controller and the temperature controller was immediately reset to 70° C. The temperature of the circulating liquid was observed to drop sharply and then slowly rise to 70° C. The reaction was maintained at 70° C for 4.5 hours with constant mechanical stirring at 120 rpm then cooled to ambient temperature by resetting the temperature controller to 20° C.

The mechanical stirrer was removed and the neck stoppered with a #59 suba seal. A large polyethylene beaker was filled with 12 L of hexanes and stirred with an IKA® Eurostar power control-visc overhead mechanical stirrer at a rate of 120 rpm. The reaction flask was then pressurized with nitrogen to 3 psi. and transfered through a polyethylene hose connected to 8 x 20 guage stainless steel syringe needles into the stirred reagent grade hexane (VWR). The mother liquor was siphoned off of the resulting precipitate and the solids were washed with 3 x 2 L of reagent grade hexane (VWR). As much solvent as possible was removed by vacuum through a polyethylene frit before the solids were transferred to a 3 L recovery flask and dried for 17 hrs. using a rotary evaporator at a bath temperature of 40° C and ultimate vacuum of -30 in. Hg.

Functionalization and Purification

A quantity of the dried polymer from the backbone reaction (25.00 g) was weighed into a 200 mL schlenk flask along with a magnetic stir bar. The flask and solids were purged 3 times to 100 mTorr and flushed with nitrogen before 100 mL of anhydrous reagent grade pyridine (Aldrich) were added via canula. The mixture was stirred for 60 minutes until the solids had completely dissolved and a homogenous solution was achieved. Redistilled, purified MAH (572 $\mu$L, methacrylic anhydride, purchased from Aldrich) was added dropwise to the stirred solution using an Eppendorf® pipette under a blanket of nitrogen. The flask was covered with aluminum foil and allowed to stir for 17 hr.

The reaction mixture was next diluted with 300 mL of anhydrous reagent grade ethanol (VWR) and the flask was rinsed with 3 x 100 mL of anhydrous reagent grade ethanol. The combined solution and rinsings were collected in a 1 L polyethylene flask which was shaken until a homogenous solution was achieved. A corner of the polyethylene flask was punctured 4 times with a 20 guage stainless steel syringe needle. The ethanol solution was then squeezed through the puncture holes into 3.5 L of well stirred reagent grade hexane (VWR). The mother liquor was siphoned off of the resulting precipitate and the solids were washed with 2 x 1 L of reagent grade hexane (VWR). As much solvent as possible was removed by vacuum through a polyethylene frit before the solids were transferred to a 1 L recovery flask and dried for 2 hr. on a rotary evaporator at ambient temperature and an ultimate vacuum of-30 in. Hg.

The total mass of dried, crude solids obtained from the functionalization reaction was dissolved in 250 mL of anhydrous reagent grade ethanol with magnetic stirring. Dowex® 2030 anhydrous acid ion exchange resin (25 g) were then added to the mixture and stirred for 3 minutes. The solution was then filtered through a glass fiber filter (VWR, 1 μm particle retention size) using a partial vacuum. The flask and resin were rinsed with 5 x 50 mL of reagent grade ethanol (VWR) and the rinsings were also filtered through the glass fiber filter. The combined solution and rinsings were collected in a 500 mL polyethylene flask which was shaken until a homogenous solution was achieved and punctured 4 times in one corner using a 20 guage stainless steel syringe needle. The ethanol solution was then squeezed through the puncture holes into 3 L of well stirred reagent grade hexane (VWR). The mother liquor was siphoned off of the resulting precipitate and the solids were washed with 2 x 1 L of reagent grade hexane (VWR). As much solvent as possible was removed by vacuum through a polyethylene frit before the solids were transferred to a 1 L recovery flask and dried for 17 hrs. on a rotary evaporator at ambient temperature and an ultimate vacuum of -30 in. Hg.

Example 2

The components in Table 1 were weighed together in a syringe to obtain a total batch size of 10 grams. The ingredients were stirred for about 5 minutes until a homogeneous mixture was obtained. This mixture was then pumped into a new syringe and centrifuged for 5 minutes at about 3000 RPM. A plunger was then inserted into the syringe to ready it for dispensing.

Table 1

| Component | Wt % |
|---|---|
| Prepolymer | 0.2 |
| Pheniramine[a] | 0.55 |
| Irgacure 1850[b] | 39.8 |
| PEG 200 | 60 |
| [a] 0.82% pheniramine in PEG 200 solution (polyethylene glycol 200 (PEG 200) prepared by dissolving 0.1245g of pheniramine in 14.9980g of PEG 200) [b] 10% Irgacure 1850 (Ciba Specialty Chemical), in PEG 200 solution | |

In a glove box filled with 50°C air, about 70 mg of the compounded prepolymer was dosed into front curve lens molds made from Zeonor (commercially available from Zeon, Corp.). Back curve lens molds also made from Zeonor were placed onto the front curves to form the lens mold cavity. The front curve/back curve mold assembly was sealed with a force of about 5 pounds (2.27 kg) while heating the assembly to about 70°C. The lens mold assemblies were irradiated for 60 seconds using a Xenon lamp source having an intensity of abou5t 30 mW/cm2. The lens mold assemblies were then pried open by hand. Excess cured material that was not part of the lens was discarded with the back curve.

Example 3

Five of the front curves with the lenses adhered thereto from Example 2 were dosed with 200 uL per lens of packing solution (0.185 weight % of sodium borate, 0.926 weight % boric acid and 98.89 weight % water). After 10 minutes, the lenses and the packing solution were transferred in to glass vials using plastic tweezers. These lenses were dosed with 2 mL packing solution, sealed with white silicone stoppers and aluminum caps and autoclaved upright once at 121°C for 30 minutes. The lenses were extracted for two hours by soaking individually in 20 mL of 0.1 % trifluororacetic acid in water. The extracts and packing solution were analyzed (100 uL per injection) on an HPLC equipped with Restek Ultra IBD (3um, 150x4.6 mm) column, and a UV detector. The mobile phase was a mixture of 90 parts 0.1 % TFA in water and 10 parts methanol. The integrated area for the pheniramine peak was used to quantitate samples against a calibration curve. This was repeated for three samples. The average amount of pheniramine detected in the packing solution of the three samples tested was 10.3 ± 1.3 ug.

Example 4

Five of the front curves with the lenses adhered thereto from Example 2 were released and leached in 1 L of deionized water with 800 ppm Tween 80 for 30 minutes at 45±5°C. The lenses were swabbed out of the deionized water/Tween 80 solution, blotted with a lint free paper towel, placed in separate glass vials and dosed with 2.2 ml of packing solution (0.185 weight % of sodium borate, 0.926 weight % boric acid and 98.89 weight % water), sealed with white silicone stoppers and aluminum caps and autoclaved upright once at 121 °C for 30 minutes. The amount of pheniramine in lenses was analyzed as in Example 3. The average amount of pheniramine detected in the packing solution of the three samples tested was 2.4 ±0.1 ug. Accordingly, lenses made according to the present invention retained four times more therapeutic agent than lenses which were released and leached with large volumes (greater than about 500 mL) of solution.

Example 5

Prepolymers of hydroxyethyl methacrylate (HEMA) and methacrylic acid (MAA) having a weight average molecular weight of 100 kD, a Pd of 1.7, and an average degree of functionality of 1.9% were made as described below.

A 5-liter glass reactor equipped with a stirrer, temperature control and a jacket for cooling and heating was charged with a mixture of the following chemicals

Table 2

| Component | Wt (gms) |
|-----------|----------|
| Ethanol | 2708.4g |
| HEMA | 291.95g |
| MAA | 5.96g |
| Norbloc | 2.92g |
| Blue HEMA | 0.0602g |
| TMPTMA | 0.30g |

The reactor temperature was raised to 68°C, and 2.11 g 2,2'-Azobis(2-Methylbutyronitrile) (AMBN) was added. The AMBN dissolved and the reactor was blanketed with a slow stream of nitrogen. The temperature was held at 68°C for 20 hours.

Five 1-liter jars with screw lid equipped with magnetic stirring bars were prepared and the crude product was poured into the jar, 600g in each. The solution was heated to 60°C in a water bath while constantly stirring with a magnet stirrer. Then, 48.0g heptane (8%) was added and the solution re-heated to 60°C. The stirring was stopped and the jars placed in a water bath at 60°C. The temperature was ramped down to 24°C over 20 hours. The top phase was now a clear fluid and the bottom phase was semi solid. The top phase was the biggest (about 80% of total jar) but with low polymer solids content (around 1.9-2.2%).

The top phases in each jar were discarded and the bottom phases were redissolved in aqueous ethanol in order to obtain 2125g of polymer solution characterized by the following: 12% solids and 3% water.

This solution was spray dried using a Mini Spray Dryer B-290, equipped with an Inert Loop, an Outlet Filter, and a High Performance Cyclone at the following parameters:

Table 3

| Inlet temp. | Outlet temp. | Spray flow | Aspirator | Pump |
|-------------|--------------|------------|-----------|------|
| 85°C | 50°C | 30mm | 78% | 32% |

This resulted in 250g of a fine white, fluffy powder that was about 97% dry. The powder was transferred to a number of 1 liter flasks (about 77 grams in each) equipped with a magnetic stirring bar. The flasks were vacuum treated overnight at 100 - 130 °C at vacuum pressure of less than 30mbar to further dry out the material.

The Spray dried backbone powder was filled into weighed flasks and vacuum treated overnight at 100 -130°C

8

at vacuum pressure less than 30 mbar. The next morning, the vacuum was broken with a dry argon atmosphere, and the flasks were transferred to a box with a controlled dry nitrogen atmosphere. The gross weight of the flasks was determined after cooling off.

300 mL NMP (N-methylpyrrolidone purum absolute, from Fluka) was added to fully dissolve the powder and the flasks were checked for homogeneity. The desired amount of MAH (methacrylic anhydride 98% pure) was weighed in a 50cc cylindrical glass container and 50 mL NMP was added to dilute the MAH before transfer. Another 50 mL NMP was used to flush the glass container, to ensure full transfer. The necessary amount of triethylamine (puriss grade, from Fluka) was added directly using a finn pipette. Lids were tightened and sealed with tape and nitrogen flow was turned off. The reaction was allowed to run for about 40 hours.

The polymer produced above was purified as follows. For purification of one of the flasks containing about 75g polymer dissolved in 400mL NMP two 5-liter glass beakers were charged each with 4 liters of DI water, 30mL fuming HCl (hydrochloric acid) and a magnetic stirring bar. The functionalized product from the previous reaction was poured gradually into the beakers, 200mL in each at a rate of about 10mL/sec. A precipitation occurred and the aqueous phase was removed. The remaining swollen polymer was redissolved in 300mL ethanol.

Two more 5-liter glass beakers were charged each with 4 liters of DI water and a magnetic stirring bar. The polymer / ethanol solution was poured into the two 5-liter glass beakers and a precipitation occurred again. The aqueous phase was removed and fresh DI water was added in order to further extract residual HCl from the swollen polymer. After about 12 hours the aqueous phase was removed and the weight of the swollen polymeric material was determined.

The swollen polymeric material was re-dissolved in ethanol to obtain a %solids content of 13.5 $\pm$0.5 %, subsequently the solution was filtrated trough a 25 mm GD/X 0.45$\mu$m Whatmann filter. The solution was spray dried using a Mini Spray Dryer B-290, equipped with an Inert Loop, an Outlet Filter, and a High Performance Cyclone. The following parameters were applied:

Table 4

| Inlet temp. | Outlet temp. | Spray flow | Aspirator | Pump |
|---|---|---|---|---|
| 79°C | 43°C | 30mm | 80% | 26% |

This resulted in about 50g of a fine white, fluffy prepolymer powder. The prepolymer powder was dried overnight at room temperature under vacuum pressure of less than 30 mbar to ready the material for use.

Example 6

The components in Table 1 below were weighed together in a syringe to obtain a total batch size of 11 grams. The ingredients were stirred for about 5 minutes at room temperature while under vacuum of about 50 mbar. until a homogeneous mixture was obtained. This mixture was then pumped into a new syringe and centrifuged for about 10 minutes at about 3000 RPM. A plunger was then inserted into the syringe to ready it for dispensing.

Table 5

| Component | Wt.% |
|---|---|
| Prepolymer | 34.45 |
| Ketotifen | 0.05 |
| CGI-403 | 0.50 |
| PEG 200 | 45.00 |
| DI water | 20.00 |

In a glove box filled with nitrogen and less than 2% oxygen at room temperature, about 70 mg of the compounded prepolymer was dosed into individual front curve lens molds made from polystyrene. Back curve lens molds made from Zeonor plastic (commercially available from Zeon, Corp.) were placed onto the front curves to form the lens mold cavity. The front curve/back curve mold assembly was sealed with a force of about 5 pounds (2.3 kg) at room temp. The lens mold assemblies were irradiated for 4 minutes using continuous wave TL-03 bulbs having an intensity of about 5 mW/cm$^2$ as measured at the lens mold using an IL1400 radiometer. The lens mold assemblies were then pried open by hand as to retain the lens on the front curve surface. The excess cured material that was not part of the lens was discarded with the back curve.

The lenses were released individually from the front curve mold using 1 ml of borate buffered saline solution containing about 800 ppm Tween 80. After equilibration for 24 hours the equilibration solution from two lenses was analyzed for Ketotifen concentration via HPLC. The averaged amount of ketotifen in the equilibration solutions was 4.6 μg.

Example 7

The two lenses from Example 6 were placed in 0.5ml of fresh borate buffered saline solution in a 37°C water bath with gentle shaking. At the following time intervals, 15 minutes, 30minutes, 1 hour, 5 hours, 24 hours, 32 hours and 48 hours the entire solution was removed and was placed in vials for HPLC analysis to check for ketotifen release over time.

The starting content of Ketotifen in the lens was confirmed by soaking two lenses in 3mls of methanol overnight to swell the lens and extract the Ketotifen. The methanol solution was analyzed by HPLC for Ketotifen concentration. The results are shown in Table 6, below.

Ketotifen concentration was analyzed on RP-HPLC using the Agilent HPLC system under the following condition:

| | |
|---|---|
| Column: | Zorbax C 18 column, 150*4.6mm |
| Method: | KF.m |
| Flow rate: | 0.8ml/min. |
| Temperature: | 40°C. |
| Detection: | UV, 235nm. |
| Injection volume: | 5ul. |

Table 6

| Time | Cumulative Ketotifen release (ug) | |
|---|---|---|
| | Sample 1 | Sample 2 |
| 15 minutes | 1.92 | 1.67 |
| 30 minutes | 3.58 | 3.60 |
| 1 hour | 4.93 | 4.92 |
| 5 hours | 5.93 | 5.56 |
| 24 hours | 5.96 | 6.41 |
| 32 hours | 6.71 | 7.03 |
| 48 hours | 7.27 | 7.48 |

**Claims**

1. A process comprising

   a. Dosing a reactive mixture comprising at least one crosslinkable prepolymer into a mold;
   b. Curing said reactive mixture to form a medical device;
   c. Contacting, said medical device with no more than about 500 mL of solution per medical device after said curing step (b); and
   d. Incorporating, prior to or during said contacting step (c), at least one therapeutic agent.

2. The process of claim 1 wherein said medical device is an ophthalmic device.

3. A process comprising

   e. Dosing a reactive mixture comprising at least one crosslinkable prepolymer into a mold;
   f. Curing said reactive mixture to form a contact lens;
   g. Contacting, said contact lens with no more than about 500 mL of solution per contact lens after said curing step (b); and
   h. Incorporating, prior to or during said contacting step (c), at least one therapeutic agent.

4. The process of claim 3 wherein said contacting step uses between about 0.005 and about 100 mL solution per lens.

**5.** The process of claim 3 wherein said contacting step uses between about 0.1 and about 50 mL solution per lens.

**6.** The process of claim 3 wherein said contacting step uses between about 0.5 and about 10 mL solution per lens.

**7.** The process of claim 3 wherein said contacting step uses between about 0.5 and about 5 mL solution per lens.

**8.** The process of claim 3 wherein said prepolymer is formed from urethanes, methacrylates, silicones, vinyl alcohol and mixtures thereof.

**9.** The process of claim 2 or 3 wherein said solution comprises water.

**10.** The process of claim 2 or 3 wherein said solution is contact lens packing solution.

**11.** The process of claim 2 or 3 wherein said solution is borate buffered saline solution.

**12.** The process of claim 3 wherein said therapeutic agent is incorporated into the reactive mixture.

**13.** The process of claim 1, 2 or 3 wherein said therapeutic agent is incorporated into the prepolymer during dosing step (a).

**14.** The process of claim 1, 2 or 3 wherein said therapeutic agent is incorporated into the prepolymer during contacting step (c).

**15.** The process of claim 1, 2 or 3 wherein said therapeutic agent is incorporated into said reactive mixture via compounding.

**16.** The process of claim 9 wherein said solution comprises a therapeutically effective amount of said therapeutic agent.

**17.** The process of claim 3 wherein said reaction mixture further comprises at least one diluent which is displaceable by said solution.

**18.** The process of claim 17 wherein said diluent is compatible with the human eye.

**19.** The process of claim 17 wherein said therapeutic agent is soluble in said diluent during curing and storage conditions.

**20.** The process of claim 17 wherein said diluent is selected from the group consisting of water, polyethylene glycol, polypropylene glycol and combinations thereof.

**21.** The process of claim 3 wherein said mold comprises a front mold half and a back mold half and at least one mold half is used to form at least a part of a contact lens package.

**22.** The process of claim 3 wherein said mold comprises a front mold half and a back mold half and at least one mold half is reusable.

**23.** The process of claim 1, 2 or 3 further comprising the step of (e) sterilizing said medical device lens after incorporating step (d).

**24.** The process of claim 23 wherein said sterilizing step is conducted via autoclaving, microwave irradiation, UV light irradiation, chemical sterilization, asceptic packaging or a combination thereof.

**25.** The process of claim 3 wherein said therapeutic agent is selected from the group consisting of pharmaceutical agents, nutraceuticals combinations thereof and the like.

**26.** The process of claim 3 wherein said therapeutic agent comprises at least one pharmaceutical agent selected from the group consisting of antihistamines, antibiotics, glaucoma medication, carbonic anhydrase inhibitors, anti-viral agents, anti-inflammatory agents, non-steroid anti-imflammatory drugs, antifungal drugs, anesthetic agents, miotics, mydriatics, immunosuppressive agents, antiparasitic drugs, anti-protozoal drugs and combinations thereof.

27. The process of claim 3 wherein said therapeutic agent comprises at least one pharmaceutical agent selected from the group consisting of acycylovir, adrenalone, aminocaproic acid, amoxicillin, amotriphene, amoxecaine, amodiaquin, antazoline, atrophine, betaxolol, bupivacaine, carbachol, carteolol, chlorampenicol, chlortetracycline, clemastine, corynathine, cromalyn sodium, cyclopentolate, demecarium, dexamethasone, dichlorphenamide, dibutoline, diclophenac, dipivefrin, ephedrine, erythromycin, ethambutol, eucatropine, fluoromethalone, gentamycin, gramicidin, homatropine, indomethacin, ketotifen, levallorphan, levobunolol, levocabastine, lidocaine, lignocaine, lomefloxacin, loratidine, medrysone, mepivacaine, methazolamide, naphazoline, natamycin, natamycin, neomycin, noradrenaline, ofloxacin, oxybuprocaine, oxymetazoline, pheniramine, phenylephrine, physostigmine, pilocarpine, polymyxin B, prednisolone, proparacaine, pyrilamine, scopolamine, sorbinil, sulfacetamide, tamoxifen, tetracaine, tetracycline, tetrahydozoline, timolol, trifluridine, tropicamide, vidarabine, and salts and mixtures thereof. In yet another embodiment the OIOC comprises at least one therapeutic agent selected from ketotifen fumarate, nor ketotifen fumarate, , 11-dihydro-11-(1-methyl-4-piperidinylidene)-5H-imidazo[2,1-b][3]benzazepine-3-carboxaldehyde (CAS# 147084-10-4, olapatadine and mixtures thereof.

28. The process of claim 3 wherein said therapeutic agent is selected from the group consisting of ketotifen fumarate, pheniramine maleate, clemastine, loratidine, 11-dihydro-11-(1-methyl-4-piperidinylidene)-5H-imidazo[2,1-b][3]benzazcpine-3-carboxaldehyde (CAS# 147084-10-4, olapatadine and mixtures thereof.

29. The process of claim 25 wherein said therapeutic agent comprises at least one nutraceutical compounds selected from the group consisting of vitamins A, D, E, lutein, zeaxanthin, lipoic acid, flavonoids, ophthalmicially compatible fatty acids and combinations thereof

30. The process of claim 3 wherein said process is conducted without an extraction step.

31. The process of claim 3 wherein said contacting step (c) comprises at least one step selected from the group consisting of releasing said lens from said mold, extracting unreacted components from said lens, hydrating said lens and combinations thereof.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 983851 A
- US 6846892 B
- US 36363902 P
- US 68468921 B
- US 6407145 B
- US 6106746 A
- WO 03003073 A
- US 5849841 A
- US 5944853 A

### Non-patent literature cited in the description

- **ALMDAL, K.** Absolute Molar Mass Distribution Determination by Size Exclusion Chromatography. Synthesis of Narrow Molar Mass Distribution Polymers. Characterization of the Molar Mass Distribution of Poly(2-Hydroxyethyl Methacrylate) by Size Exclusion Chromatography with Coupled Refractive Index and Low Angle Laser Light Scattering Detection. *Risø-M-2787,* 1989, vol. 1, 141